# EUROPEAN PATENT APPLICATION

(11) **EP 1 723 960 A1**
(43) Date of publication of application: **22.11.2006**
(21) Application number: 05010645.9
(22) Date of filing: 17.05.2005
(51) Int. Cl.: A61K 36/00, A61K 36/18, A61P 3/00, A61P 17/02

(54) **Botanical compositions having oral insulin-like hypoglycemic activity, with hypolipidemic and antimicrobial activities**

(71) Applicant: Jesadanont, Sukanya, Chatuchak, Bangkok 10900 (TH); Jesadanont, Apijade, Chatuchak, Bangkok 10900 (TH); Jesadanont, Mongkol, Chatuchak, Bangkok 10900 (TH); Wijitphan, Kamol, Chatuchak Bangkok (TH)
(72) Inventor: Jesadanont, Sukanya, Chatuchak, Bangkok 10900 (TH); Jesadanont, Apijade, Chatuchak, Bangkok 10900 (TH); Jesadanont, Mongkol, Chatuchak, Bangkok 10900 (TH); Wijitphan, Kamol, Chatuchak Bangkok (TH)
(74) Representative: von Willich, Werner

(57) **Abstract**

Botanical compositions are described having oral insulin-like hypoglycemic activity with hypolipidemic and antimicrobial activity for maintaining good health in normal person and diabetic person comprising water extract of medicinal plants, dried leaves of *Malvastrum coromandelianum* and/or dried stems of *Coscinium fenestratum.* The extracts are used separately or in combination of fixed amount or varied amounts, as herbal beverage or in different appropriate pharmaceutical preparations.

## Description

### Field of Invention:

This invention relates to botanical composition for maintaining a healthy condition in normal people and treatment against infections including providing blood sugar and lipid lowering activity in diabetic patients and the process for preparing the same.

### Background of the Invention:

At present, people all around the world turn more and more back to nature as it is now realized that there are still a lot of very good things the mother nature has given to mankind yet has never been explored or brought to use like medicinal herbal treatment to treat cases of hard-to-cure diseases such as cancer-the disease of various origins, immune-related diseases and even AIDS**.** The patients of these hard-to-cure diseases very often died very soon because of the radical treatment and the side effects of the westernized medication while the oriental way of treatment offers a more gentle, mild yet effective choice of medicinal herbs which possesses the so-called 'Yin-Yang' curing action. That is to say, while an agent in the medical recipe helps to suppress the evil or deteriorating causes in the body, the other agent(s) in the same recipe would also promote the well-being of the good cells or healthy elements of such a person, U.S. Patent No.6,485,759B2. This is obviously shown in many circumstances and the contrary is clear when a patient has been treated using only the western scientific way of treatment where a synthetic agent used mostly suppresses the causative agent of the disease as well as the host good cells, since the western way of treatment is quite "non-selective". Thus presently, even the renowned medical schools pay greater attention to the oriental way of treatment with the hope to reach the best and optimized way combined for treatment of the symptom of a patient.

The invention described here are pharmaceutical products of medicinal herbal origin for maintaining good health in a healthy person as well as in diabetic patient and process for preparing of the product. As it is well-known that sugar especially glucose although regarded as a major source of energy and as structural elements in the body, too much of sugar intake leads to numerous diseases such as hyperlipidemia especially hypertriglyceridemia as the carbon component of glucose is very efficiently converted to fatty acids and thus to triglyceride leading to severe heart diseases and strokes which are even more dangerous than hypercholesterolemia in a normal individual. More serious is in diabetic patients whose high level of glucose in blood resulting in too many deteriorating complications occurred probably through glycosylation of membrane such as diabetic retinopathy, nephropathy and more. Keeping blood glucose at normal level has been shown to prevent efficiently such complications at a great extent. Most important of all is that even very small wound can always lead to death due to serious infections or resulting in lower extremities amputation (LEA) surgery and thus disability of the diabetic patients. It would be thus most desirable to have a medicinal composition possesses both anti-infectious and hypoglycemic activity including hypolipidemic activity. The invention described presently offers a most cost-effective formulation for maintaining good health in diabetic patients as well as in normal people and perhaps those with immune-compromised to a certain extent since the preparation has very good activity against the pus-forming microbe- *Staphylococcus aureus* of both methicillin-sensitive and methicillin-resistant strains, and a few pathogenic microorganisms including *Salmonella typhimurium.* Moreover, the plant extract composition possesses very good hypoglycemic and hypolipidemic activity. Drinking water extract of *Malvastrum coromandelianum* caused within 7 days very good healing of severe ulcerative wound in a diabetic patient who was about to undergo leg amputation surgery and the patient could avoid leg surgery thereafter.

### Summary

Botanical compositions are described having oral insulin-like hypoglycemic activity with hypolipidemic and antimicrobial activity for maintaining good health in normal person and diabetic patients comprising water extract of medicinal plants, i.e., water extract of dried leaves of *Malvastrum coromandelianum* (Linn.) Garcke, with or without the combination with water extract of dried stems of *Coscinium fenestratum* (Gaerth.) Colebr. The pharmaceutical preparations are decoction, solution, granules, tablet, capsule, reconstitute preparation or any appropriate dosage forms. The two extracts may be used separately or in combination of fixed amount or varied amounts, as herbal beverage or in different pharmaceutical preparations.

### Brief Description of the Drawing

FIG. 1 illustrates hypoglycemic effect of water extract from dried leaves of *Malvastrum coromandelianum* (Linn.) Garcke, fed in normal male Wistar rats, 100-125 grams body weight;
FIG. 2 illustrates potent hypoglycemic effect of single oral doses of water extract from dried leaves of *Malvastrum coromandelianum* (Linn.) Garcke, in normal male streptozotocin-induced diabetic rats, at 10-100 mg/kg body weight compared to that of insulin injection at 5 IU/kg body weight.

### Detailed Description of the Invention

The two plant extracts used in this invention are prepared by extracting aerial parts, preferably, leaves, stems and branches of *Malvastrum coromandelianum* (Linn.) Garcke, Malvaceae and dried stems of *Coscinium fenestratum* (Gaerth.) Colebr., Menispermaceae ; separately with water. The parts of plant used may be fresh or dried at 60 degree Celsius until constant dry weight is obtained. The dry ingredient is ground and put through a sieve to obtain powder of uniform particle size. Water is added to cover the powder using 100 liters for 10 Kg dry weight of powder and the mixture is heated until boiled for five minutes and simmered at 80 degree Celsius for the next 7-8 hr. The filtrate is collected and the residual powder is further boiled in 50 liters water and simmered in similar manner once or twice. All filtrates are pooled and dried either spray-dried, freeze-dried or concentrating-dried to obtain dried powder extract of the ingredient referred to as water extract of *M. coromandelianum* (Pinkish-light brown) or *C. fenestratum* (yellowish-brown). Generally, a yield of 10 % dry weight is obtained for each extraction. Alternatively, aqueous organic solvent such as ethyl alcohol and water mixture may also be used instead of water although not quite as good, where a similar extraction process is used to extract the plant ingredient to give the dried plant extract used in the present invention. The constituents in the dried plant extract are identified and quantitatively analyzed using conventional High Performance Liquid Chromatography (HPLC). The same method is used to quantify the amount of plant constituent to be manufactured the pharmaceutical preparations. Furthermore, in Thailand, the country of plant origin where these plants are grown and harvested, - the weather does not vary much during the year. Thus, there is not much difference between the crops harvested at different time of the year regarding the quality and the quantity of the constituents of the plant when analyzed by HPLC.

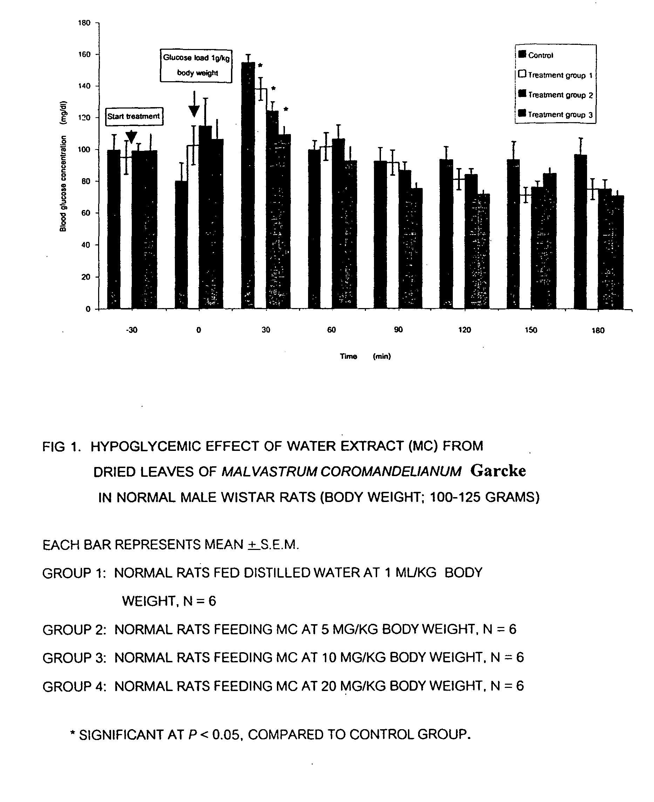

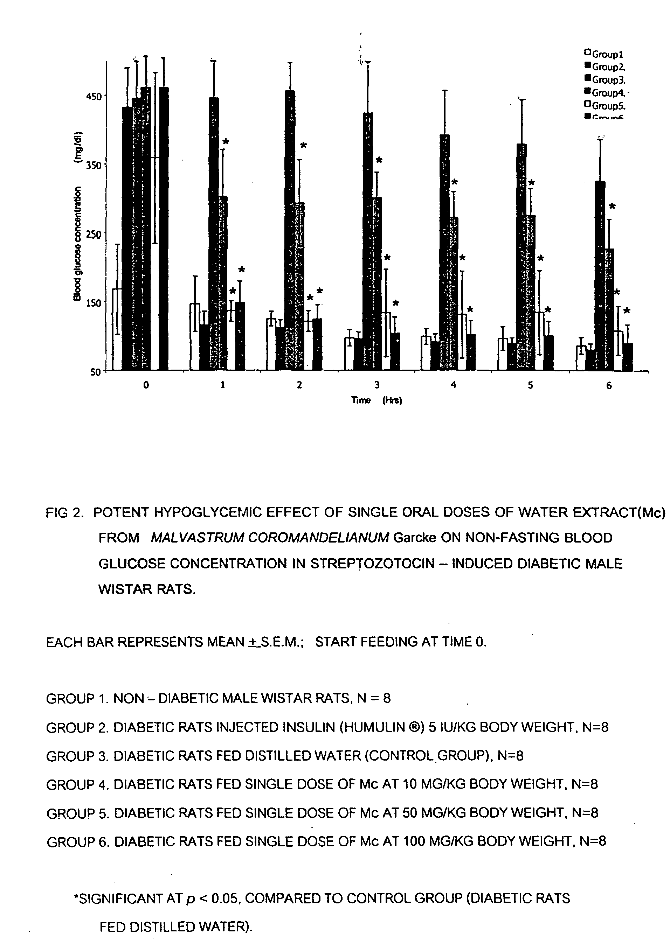

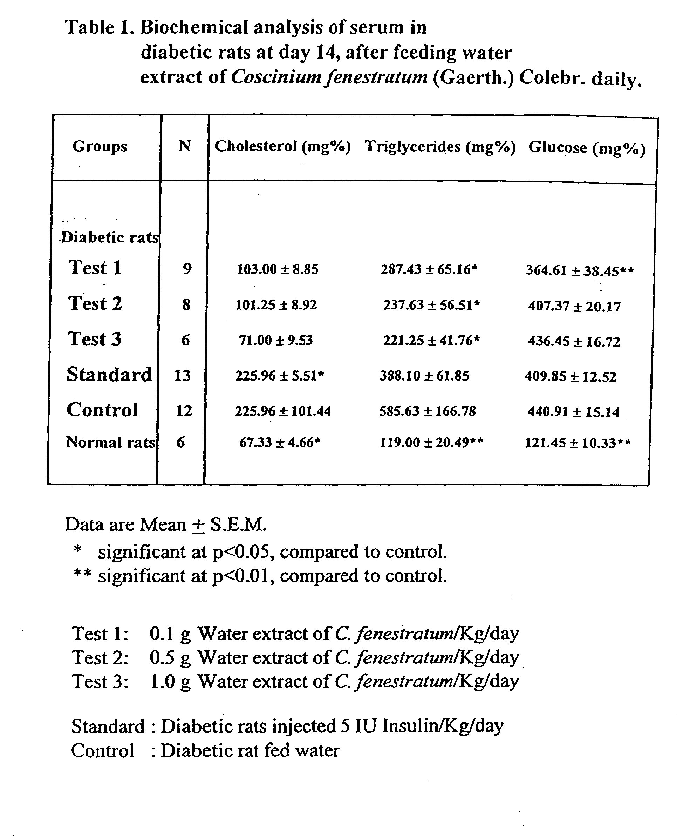

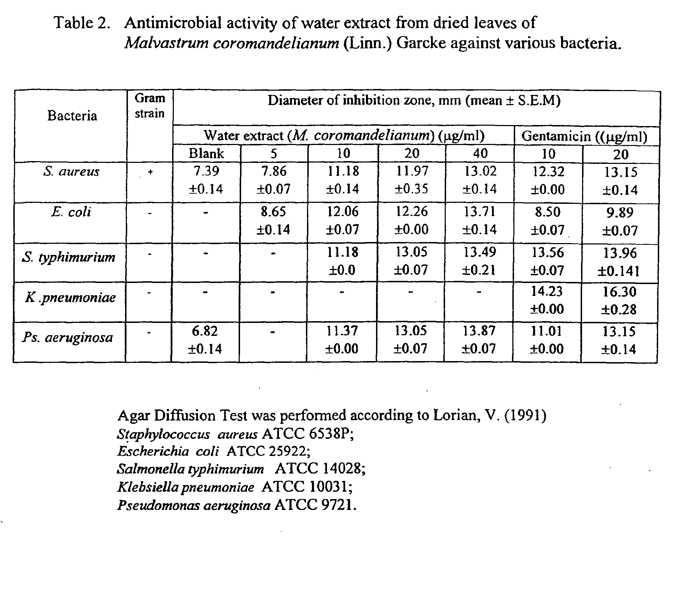

The dried plant extract is ground to fine powder and sieved to obtain uniform powder size and used for pharmaceutical preparation. The extract of plant *C. fenestratum* possesses good hypoglycemic and hypolipidemic activity in streptozotocin-induced diabetic rats (Table 1), while that of *M. coromandelianum* possesses very good hypoglycemic (Fig. 1) in normal rats and in streptozotocin-induced diabetic rats (Fig. 2) with good antimicrobial activity (Table 2) against several pathogenic microorganisms where Agar Diffusion Test was performed according to Lorian, V. Ed. (1991) Antibiotics in Laboratory Medicine, 3rd ed. The pathogenic microbes inhibited by the crude water extract of *M coromandelianum* are *Staphylococcus aureus,* and *Salmonella typhimurium.* The glucose tolerance test showed very good hypoglycemic activity of the *M coromandelianum* extract at 5 mg/kg body weight in normal rats as suppression of blood glucose level is distinct. Increasing amount of extract to 10 and 20 mg/kg body weight results in correspondingly increase in suppression of blood glucose level (Fig. 1). In diabetic rats, feeding the water extract of *M coromandelianum* at 50-100 mg/kg body weight suppresses the blood glucose from over 400 mg/dL down to a level comparable to that of insulin injection 5 IU/kg which is slightly over 100 mg/dL (Fig. 2) and sustains such activity for at least 5 hours, where increasing the extract further to a dose of 500 mg/kg does not suppress the blood glucose any further significantly. Thus, the extract acts orally just like insulin which would be most desirable for all diabetic patients since the preparation does not need to be injected. Just taking easily orally would help bringing down the blood glucose level effectively.

These medicinal herb extracts can thus be used either singly or in combination in several ways. Firstly, it can be used as tea where dried ground coarse powder mixture of plant parts can be subjected to decoction by being boiled gently in water for a few minutes and the water extract is drunk on and off during the day. Alternatively, the coarse powder of dried ground leaves and stem can be packed in small bag made of heat-stable durable material with appropriate pore size to allow active ingredients of the plant to dissolve in hot water to be drunk as tea while retaining all the insoluble plant part within the bag. Secondly, the dried powder or granules of water extract can be served as hot instant beverage with or without flavoring or sweetening agent (not glucose nor sucrose nor energy-providing substances, i.e. non-sugar, non-carbohydrate, no-calories sweetener) which can be either aspartame or stevia, etc. The extract can be used conveniently as instant tea by preparing as granule using any appropriate pharmaceutical binder, preferably, natural gums such as acacia, tragacanth, agar, starch mucilage, carrageenan and/or any appropriate synthetic binder used singly or as combination. Thirdly, the granule or the extract powder can be formulated to be used as solid pharmaceutical preparations, preferably, capsule or tablet to be given orally in various doses, 1 - 3 times daily depends on the health condition of a person. Other pharmaceutical dosage forms may also be applied such as non-sugar aqueous preparation or elixir with stability of the active ingredients to be given in equivalent doses. The water extract of the plant is the best way possible to use as extract from plant since the process is the simplest with no chance of contamination of organic solvent(s) which are much more expensive and can do harm to the environment including to human since even small amount of some organic solvents such as carbon tetrachloride or chloroform can cause ill effect(s) to a person especially to the liver, as well as can be very likely carcinogenic or may cause allergy.

In preparation of extract solution from plant parts, the preparation and/or separation of an extract solution may be alternatively carried out according to a method of any scientific or pharmaceutical means such as pressing, centrifugal separation and etc.

The extract solution obtained according to the above method is alternatively condensed by means of a reverse osmosis membrane. This condensation by means of a reverse osmosis membrane has advantages that the active ingredients are preserved although most of them are quite heat-stable. The concentrate solution is further dried by vacuum freeze-drying.

The preparations of the plant extract(s) having hypoglycemic, antimicrobial, with/without hypolipidemic activities are at least any of the following examples.

### Example 1

Dried ground leaves (0.5-10.0 grams) of *M coromandelianum* is immersed under 150-800 milliliters hot water (60-90°C) for 5-10 minutes. Supernatant is decanted and drunk as herbal tea to maintain good health having blood sugar lowering activity. The water extract also has good antimicrobial activity against pathogenic microorganism, i.e. *S. aureus.*

### Example 2

Dried ground leaves (0.5-10.0 grams) of *M coromandelianum* used singly or in combination with dried ground stems (0.5-20.0 grams) of *C. fenestratum* are immersed under 150-800 milliliters hot water (60-90°C) for 5-10 minutes. Supernatant is decanted and drunk as herbal tea to maintain good health as blood sugar would be lowered to an appropriate level. The extracts in combination possess good hypoglycemic, hypolipidemic (both hypotriglyceridemic and hypocholesterolemic) and antimicrobial activities.

### Example 3

Dried ground leaves (0.5-10.0 grams) of *M coromandelianum* is packed in porous, heat-stable bag and immersed under 150-800 milliliters hot water (60-90°C) for 5-10 minutes. The bag is removed and the light yellow or brownish water extract is drunk as tea.

### Example 4

Dried ground leaves (0.5-10.0 grams) of *M coromandelianum* used singly or in combination with dried ground stems (0.5-20.0 grams) of *C. fenestratum* are packed in porous, heat-stable bag and immersed under 150-800 milliliters hot water (60-90°C) for 5-10 minutes. The bag is removed and the yellow or brownish water extract is drunk as tea all through the day.

### Example 5

### Granulation of plant water extracts

Water extract of *M coromandelianum* or water extract of *C. fenestratum,* using 0.5 - 5% binders in the group of hydrocolloids and/or natural gum such as alginate, gum, agar, carrageenan, konjak flour, acacia, or tragacanth is granulated separately by conventional process for granulation, preferably, wet granulation.

Sieve the granules through sieve No. 30, collect the granules and dry at 50 ° Celsius until constant weight is obtained, kept under air-tight condition protected from light and moisture.

### Example 6

### Herbal beverage with therapeutic properties-hypoglycemic, antimicrobial and hypolipidemic activities:

Granule of water extract of *M. coromandelianum* is dissolved in water (0.01-10.0 grams, preferably 500 mg, of water extract per 150-800 ml) singly or in combination with granule of water extract of *C. fenestratum* (0.05-25.0 grams, preferably 1,000 mg, of water extract per 150-800 ml); to be drunk on and off during the day.

### Example 7

| | |
|---|---|
| Water extract of *M. coromandelianum* | 10 -1,200 mg |
| Diluent | 75 - 175 mg |
| Lubricant | 10 - 20 mg |
| Binder | 2 - 5 mg |
| Total weight per capsule or tablet | 200 - 1,200 mg |

To be taken orally.

### Example 8

| | |
|---|---|
| Water extract of *M. coromandelianum* | 1,000 mg |
| Diluent | 175 mg |
| Lubricant | 20 mg |
| Binder | 5 mg |
| Total weight per capsule or tablet | 1,200 mg |

To be taken orally.

### Example 9

| | |
|---|---|
| Water extract of *M. coromandelianum* | 10 - 1,200 mg |
| Water extract of *Coscinium fenestratum* | 50 - 1,500 mg |
| Diluent | 75-175 mg |
| Lubricant | 10-20 mg |
| Binder | 2-15 mg |
| Total weight per capsule or tablet | 200- 1,500 mg |

To be taken orally.

### Example 10

| | |
|---|---|
| Extract of *M. coromandelianum* | 600 mg |
| Extract of *C. fenestratum* | 750 mg |
| Diluent | 130 mg |
| Lubricant | 15 mg |
| Binder | 5 mg |
| Total weight per capsule or tablet | 1,500 mg |

To be taken orally.

For all the above examples:

The diluents used are of any groups of natural or synthetic origin include: non-sweet, no or low calories, inert substance, preferably, microcrystalline cellulose, talcum, sorbitol, or corn starch, and where said diluents used may be only of one kind or mixture of several kinds in the range of 10 to 80 % by weight;

The lubricants used are of any groups of natural or synthetic origin include: mineral oil, fatty acid or its salt or vegetable oil, preferably, light mineral oil, hydrogenated vegetable oil, stearic acid, or stearate (Mg, Ca or Na), and where said lubricants used may be only of one kind or mixture of several kinds in the range of 1 to 15% by weight;

The binders used are of any groups of natural or synthetic origin include: hydrocolloids and natural gum, preferably, alginate, gum, agar, carrageenan, konjak flour, acacia, tragacanth, and where said binders used may be only of one kind or mixture of several kinds in the range of 0.1 to 10% by weight; and

The capsule used is hard gelatin capsule or the like of appropriate size. Pharmaceutical preparations of any hard or solid types is alternatively applied.

These pharmaceutical preparations are not limited to what has been described above, they are alternatively any of other conventional preparations such as solution, reconstituted non-sugar liquid preparation and more of the appropriate dosage forms. They are used to maintain good health in a normal person to keep blood sugar level not to exceed the desirable level. It is used in a diabetic person as it possesses all the desirable effects for diabetic patient, i.e. hypoglycemic effect, hypotriglyceridemic, hypocholesterolemic and good antimicrobial activity against various pathogenic microbes such as *S. aureus,* and *S. typhimurium.* It also appears to have good wound healing effect in man. The oral insulin-like activity of the preparation makes it most desirable for diabetic patients to allow taking the medicine orally safely, easily and help avoiding the infection and all the adverse effects which are well-known to be caused by injection of insulin especially as an unavoidable life-long treatment.

It will be understood that modifications may be made within the scope of this invention by one of ordinary skill in the art without departing from the spirit thereof. It is accordingly intended that all matter contained in the above description be interpreted as illustrative rather than in a limiting sense.

It is also to be understood that the following claims are intended to cover all of the generic and specific features of the invention as described herein, and all statements of the scope of the invention which, as a matter of language, might be said to fall therebetween.

## Claims

1. A herbal beverage having hypoglycemic, and antimicrobial activity against *Staphylococcus aureus,* comprises water extract in form of liquid obtained by immersing under hot water, 60-90 degree Celsius, for a predetermined period of time a plant composition consisting of dried ground leaves of *Malvastrum coromandelianum,* 0.5-10.0 grams, used singly or in combination with dried ground stems of *Coscinium fenestratum,* 0.5-20.0 grams, as coarse powder per se or coarse powder packed in porous, heat-stable bag with appropriate pore size to retain the plant residue in the bag, where the water extract is drunk as tea to maintain good health having blood sugar lowering and anti-pus activities.

2. Herbal composition having hypoglycemic, hypolipidemic and antimicrobial activity against at least *Staphylococcus aureus,* comprises water extract of dried leaves of *M. coromandelianum,* in form of powder or granule, singly or in combination with water extract of dried stems of *C. fenestratum* in form of powder or granule, in varied amounts, 0.01-10 grams and 0.05-25 grams, respectively, dissolved in water or prepared in form of liquid pharmaceutical preparation, and to be taken orally.

3. Herbal composition having hypoglycemic, hypolipidemic and antimicrobial activity against at least *Staphylococcus aureus* of claim 2, whereof water extract of dried leaves of *M coromandelianum* is used in form of powder or granule, singly or in combination with water extract of dried stems of *C. fenestratum* in form of powder or granule, in fixed amount, preferably, 500 mg and 1,000 mg, respectively, dissolved in water or prepared in form of liquid pharmaceutical preparation, and to be taken orally.

4. Composition of botanical origins formulated as appropriate solid pharmaceutical preparation(s), preferably, capsule or tablet having following ingredients in varied amounts:
| | |
|---|---|
| Water extract of *M. coromandelianum* | 10 - 1,200 mg |
| Water extract of *C. fenestratum* | 50 - 1,500 mg |
| Diluent | 75 -175 mg |
| Lubricant | 10 - 20 mg |
| Binder | 2 - 5 mg |
| Total weight per capsule or tablet | 200 - 1,500 mg |
To be taken orally, where of
said diluents used are of any groups of natural or synthetic origin include: non-sweet, low energy, inert substance, preferably, microcrystalline cellulose, talcum, or corn starch, and where said diluents used may be only of one kind or mixture of several kinds in the range of 20 to 80 % by weight;
said lubricants used are of any groups of natural or synthetic origin include: mineral oil or vegetable oil, preferably, light mineral oil, hydrogenated vegetable oil, stearic acid, or stearate (Mg, Ca or Na), and where said lubricants are used only of one kind or mixture of several kinds in the range of 1 to 15% by weight;
said binders used are of any groups of natural or synthetic origin include: hydrocolloids and natural gum, preferably, alginate, gum, agar, carrageenan, konjak flour, acacia, tragacanth, and where said binders are used only of one kind or mixture of several kinds in the range of 0.1 to 10% by weight; and
said pharmaceutical preparations used are hard gelatin capsule or any types of tablet or any type of appropriate solid pharmaceutical dosage forms.

5. Composition of botanical origins comprises ingredients of claim 4 in fixed amount:
| | |
|---|---|
| Water extract of *M coromandelianum* | 600 mg |
| Water extract of *C. fenestratum* | 750 mg |
| Diluent | 130 mg |
| Lubricant | 15 mg |
| Binder | 5 mg |
| Total weight per capsule or tablet | 1,500 mg |
To be taken orally.

6. Herbal composition comprises water extract of dried leaves of *M. coromandelianum,* in form of solid preparation, preferably, powder or granule, in varied amounts 0.01-10 grams, dissolved in water or and to be taken orally.

7. Composition of botanical origin comprises herbal component of claim 6 being formulated as appropriate solid pharmaceutical preparations, preferably, capsule or tablet, having following ingredients in varied amounts:
| | |
|---|---|
| Extract of *M. coromandelianum* | 10 - 1,200 mg |
| Diluent | 75 - 175 mg |
| Lubricant | 10 - 20 mg |
| Binder | 2 - 5 mg |
| Total weight per capsule or tablet | 200 - 1,200 mg |
To be taken orally; where of
said diluents used are of any groups of natural or synthetic origin include: non-sweet, low energy, inert substance, preferably, microcrystalline cellulose, talcum, or corn starch, and where said diluents are used only of one kind or mixture of several kinds in the range of 20 to 80 % by weight;
said lubricants used are of any groups of natural or synthetic origin include: mineral oil or vegetable oil, preferably, light mineral oil, hydrogenated vegetable oil, stearic acid, or stearate (Mg, Ca or Na), and where said lubricants are used only of one kind or mixture of several kinds in the range of 1 to 15% by weight;
said binders used are of any groups of natural or synthetic origin include: hydrocolloids and natural gum, preferably, alginate, gum, agar, carrageenan, konjak flour, acacia, tragacanth, and where said binders are used only of one kind or mixture of several kinds in the range of 0.1 to 10% by weight; and
said pharmaceutical preparations used are hard gelatin capsule or any types of tablet or any type of appropriate solid pharmaceutical dosage forms.

8. Composition of botanical origin comprises herbal component of claim 7 being formulated as appropriate solid pharmaceutical preparations, preferably, capsule or tablet, having following ingredients in fixed amount:
| | |
|---|---|
| Extract of *M coromandelianum* | 1,000 mg |
| Diluent | 175 mg |
| Lubricant | 20 mg |
| Binder | 5 mg |
| Total weight per capsule or tablet | 1,200 mg |
To be taken orally.

9. Use of pharmaceutical preparation of claim 5 for at least hypoglycemic, antibacterial and hypolipidemic activities.

10. Use of pharmaceutical preparation of claim 7 for at least hypoglycemic and antibacterial activities.
